# EUROPÄISCHE PATENTSCHRIFT

(11) **EP 1 907 038 B1**
(45) Veröffentlichungstag und Bekanntmachung des Hinweises auf die Patenterteilung: **02.05.2018**
(21) Anmeldenummer: 06754148.2
(22) Anmeldetag: 06.06.2006
(51) Int. Cl.: A61M 16/00, A61M 16/12, B01D 53/22

(54) **VORRICHTUNG UND VERFAHREN ZUR AUFBEREITUNG VON GASGEMISCHEN**
DEVICE AND METHOD FOR PREPARING GAS MIXTURES
DISPOSITIF ET PROCEDE DE PREPARATION DE MELANGES GAZEUX

(30) Priorität: 14.07.2005 DE 102005032977
(43) Veröffentlichungstag der Anmeldung: 09.04.2008
(73) Patentinhaber: Schmidt, Klaus Michael, Halifax, Nova Scotia B3H 4S9 (CA)
(72) Erfinder: Schmidt, Klaus Michael, Halifax, Nova Scotia B3H 4S9 (CA)
(74) Vertreter: Weickmann & Weickmann PartmbB
(86) Internationale Anmeldenummer: PCT/EP2006/005376
(87) Internationale Veröffentlichungsnummer: WO 2007/006377

(56) Entgegenhaltungen:
- EP-A- 0 901 985
- WO-A-00/33949
- WO-A-03/092778
- WO-A1-01/07108
- WO-A1-2004/060459
- DE-A1- 19 545 598
- US-A- 4 622 976
- US-A1- 2002 104 542
- US-B1- 6 236 041
- US-B1- 6 471 747

## Beschreibung

### I. Anwendungsgebiet

Die Erfindung betrifft die Aufbereitung von Gasgemischen, insbesondere von Beatmungsgasen für beatmete Patienten.

### II. Technischer Hintergrund

Das bedarfsgerechte zur Verfügung stellen von Gasgemischen, bei denen eine vorgegebene Konzentration der Einzelkomponenten eingehalten wird, ist technisch machbar.

Wenn das gesamte Gasgemisch einem Verbraucher nicht in einem geschlossenen, sondern in einem offenen Kreislauf zur Verfügung gestellt wird und die Ziel-Fraktion des Gases sich dabei teilweise verbraucht, der Rest der Ziel-Fraktion jedoch wieder verwendet oder zumindest gesammelt werden muss, wird dies bereits sehr viel aufwändiger, vor allem in Abhängigkeit von den zu bearbeitenden Mengen und davon, um welche Ziel-Fraktion im Vergleich zu den restlichen Fraktionen es sich handelt.

Wenn die Ziel-Fraktion ein Edelgas, beispielsweise Xenon, ist, wird die Handhabung dadurch eher aufwändiger.

Im medizinischen Bereich hat sich jedoch herausgestellt, dass gerade eine Zuführung von Xenon in den Atemkreislauf von beatmeten Patienten für die Sedierung und auch den Schutz der Gehirnfunktionen des Patienten förderlich ist.

Dem Nutzen diese Erkenntnis steht jedoch in der Praxis erschwerend entgegen, dass
- Xenon in der benötigten Menge relativ teuer ist bei einem Literpreis von ca. 15 USD und einem Bedarf pro Patient von 6 Litern pro Beatmungsminute, und
- aufgrund des Kostendruckes bei der Anschaffung neuer Beatmungsgeräte, die eine Xenonzufuhr gestatten, gezögert wird.

Aus der WO 03/092778A ist ein Beatmungsgerät mit einem Intubationsschlauch, einer Gasmischkammer und einem Druckerzeuger bekannt, wobei zwischen Intubationsschlauch und Mischkammer ein Selektionselement bzgl. der Ziel-Fraktion des Beatmungszurückgases von dessen restlichen Fraktionen vorhanden ist und einem Zielzuführanschluss, der mit einem gesteuerten Zufuhrregler mit einem Ziel-Fraktion- Reservoir in Verbindung besteht.

Die US 6471747 B1 beschreibt ein Verfahren und eine Vorrichtung zur Zuführung und Wiedergewinnung von Gasen in der Anästhesie, wobei das verbrauchte Gas vorzugsweise im Wege eines medizinischen Verfahrens rückgewonnen wird.

Die WO 00/33949 A betriff eine Membran zum Abtrennen von Xenon aus Sauerstoff und Stickstoff und ein Verfahren zur Verwendung desselben. Im Wesentlichen werden Gastrenn-Membranen einer spezifischen Zusammensetzung beschrieben, nicht jedoch, dass diese Gastrenn-Membranen bei der Rückgewinnung vom verbrauchten Anästhesiegas eingesetzt werden sollen.

Aus der WO 01/07108 A1 ist ein auf Xenon basierendes Anästhesie-Verfahren bekannt, bei dem ein Intubationsschlauch, ein Gasmisch, ein Druckerzeuger und ein Selektionselement eingesetzt werden.

Aus der WO 03/092778A ist ein Beatmungsgerät mit einem Intubationsschlauch, einer Gasmischkammer und einem Druckerzeuger bekannt, wobei zwischen Intubationsschlauch und Mischkammer ein Selektionselement bzgl. der Ziel-Fraktion des Beatmungszurückgases von dessen restlichen Fraktionen vorhanden ist und einem Zielzuführanschluss, der mit einem gesteuerten Zufuhrregler mit einem Ziel-Fraktion-Reservoir in Verbindung besteht.

Die US 6471747 B1 beschreibt ein Verfahren und eine Vorrichtung zur Zuführung und Wiedergewinnung von Gasen in der Anästhesie, wobei das verbrauchte Gas vorzugsweise im Wege eines medizinischen Verfahrens rückgewonnen wird.

Die WO 00/33949 A betriff eine Membran zum Abtrennen von Xenon aus Sauerstoff und Stickstoff und ein Verfahren zur Verwendung desselben. Im Wesentlichen werden Gastrenn-Membranen einer spezifischen Zusammensetzung beschrieben, nicht jedoch, dass diese Gastrenn-Membranen bei der Rückgewinnung vom verbrauchten Anästhesiegas eingesetzt werden sollen.

Aus der WO 01/07108 A1 ist ein auf Xenon basierendes Anästhesie-Verfahren bekannt, bei dem ein Intubationsschlauch, ein Gasmisch, ein Druckerzeuger und ein Selektionselement eingesetzt werden.

Die WO 02004/060459 A1 beschreibt eine Gasregelungseinrichtung, mittels der Stickstoff aus einem Gasfluss, wie ausgeatmetem Atem, welches ein Anästhesiegas in einem Anästhesiekreislauf enthält, wiedergewonnen wird.

Aus der US 2002/0104542 A1 ist eine Anästhesie-Filteranordnung bekannt, mit der das Gas mittels eines Filters zurückgehalten werden kann.

Die US 6236041 B1, die der DE 19545598 A1 entspricht, offenbart eine Vorrichtung zur analytischen Bestimmung und Regulierung der Zusammensetzung von Narkosegasen, die eine massenspektrometrische Sonde, eine Xenonquelle, einen Messkanal für die Überwachung der Zusammensetzung des verbrauchten Narkosegases, einen Messkanal zur Überwachung der Zusammensetzung des im Beatmungskreislauf einzuspeisenden Narkosegases, ein Massenspektrometer und eine Aufbreitungsanlage für verbrauchte Narkosegas enthält.

Die WO 02004/060459 A1 beschreibt eine Gasregelungseinrichtung, mittels der Stickstoff aus einem Gasfluss, wie ausgeatmetem Atem, welches ein Anästhesiegas in einem Anästhesiekreislauf enthält, wiedergewonnen wird.

Aus der US 2002/0104542 A1 ist eine Anästhesie-Filteranordnung bekannt, mit der das Gas mittels eines Filters zurückgehalten werden kann.

Die US 6236041 B1, die der DE 19545598 A1 entspricht, offenbart eine Vorrichtung zur analytischen Bestimmung und Regulierung der Zusammensetzung von Narkosegasen, die eine massenspektrometrische Sonde, eine Xenonquelle, einen Messkanal für die Überwachung der Zusammensetzung des verbrauchten Narkosegases, einen Messkanal zur Überwachung der Zusammensetzung des im Beatmungskreislauf einzuspeisenden Narkosegases, ein Massenspektrometer und eine Aufbreitungsanlage für verbrauchte Narkosegas enthält.

### III. Darstellung der Erfindung

### a) Technische Aufgabe

Es ist daher die Aufgabe gemäß der Erfindung, eine Vorrichtung und ein Verfahren zu schaffen, welches die Applikation einer zusätzlichen Gas-Fraktion, speziell von Xenon, im Rahmen eines Gasgemisches, beispielsweise eines Beatmungsgases für Patienten, besonders einfach und unter möglichst geringen Verbrauchsverlusten an Xenon gestattet.

### b) Lösung der Aufgabe

Diese Aufgabe wird durch die Merkmale in den Ansprüchen 1 und 13 gelöst. Vorteilhafte Ausführungsformen ergeben sich aus den Unteransprüchen.

Durch einen steuerbaren Zufuhrregler für die Zielfraktion zur Gemischaufbereitung des Beatmungsgases kann der Anteil der Zielfraktion, beispielsweise Xenon, im Beatmungsgas bedarfsgenau eingestellt werden.

Sofern der Rest des Beatmungsgases noch kein Xenon enthielt, weil beispielsweise aus der Umgebungsluft unter Zumischung von Sauerstoff erstellt, ist auch kein Sensor zur Ermittlung des bisherigen Gehaltes an der Zielfraktion notwendig. Falls jedoch das vom Patienten ausgeatmete Beatmungs-Rückgas im Kreis geführt und wieder als Beatmungsgas, gegebenenfalls unter Zusatz von frischem Sauerstoff, verwendet wird, muss zuvor der bereits im Rückgas enthaltene Anteil der Zielfraktion mittels eines Sensors ermittelt und bei der zusätzlichen Zugabe dieser Zielfraktion berücksichtigt werden.

Unabhängig davon ist im Gasweg des Beatmungsrückgases, also zwischen Intubations-Schlauch und entweder dem offenen Auslass für das Beatmungsrückgas oder der Rückführung in die Gasmischkammer für die Aufbereitung des neuen Beatmungsgases, ist ein Selektionselement vorhanden, um die Zielfraktion wiederverwerten zu können:
Dies kann eine aktiv wirkende Trennvorrichtung sein, die die Zielfraktion vom Rest des Gasgemisches trennt, falls das Rückgas an die Atmosphäre entlassen werden soll. Dies könnte eine Zentrifuge sein, oder eine einfache Membran, die alle anderen Komponenten des Rückgases an die Umgebung durchtreten lässt, nicht jedoch die Zielfraktion.

Falls das Beatmungs-Rückgas im Kreislauf geführt und zur Herstellung eines neuen Beatmungsgases benutzt wird, kann die im Beatmungs-Rückgas noch enthaltene Zielfraktion dort belassen werden, jedoch müssen für die Aufbereitung des Beatmungsgases dem dafür verwendeten Beatmungs-Rückgas unter Umständen neue, andere Fraktionen als die Zielfraktion zugesetzt werden (z. B. Sauerstoff) oder entzogen werden (z. B. Kohlendioxid).

Auch hier muss sicher gestellt werden, dass bei diesem Zusatz bzw. Abzug anderer Fraktionen die Zielfraktion nicht unerwünschterweise mit abgezogen wird.

Dies kann durch einen passiven Selektor z. B. in Form einer Membran erfolgen, durch welche hindurch andere Fraktionen als die Zielfraktion in die Mischkammer eintreten oder austreten können, jedoch die Zielfraktion nicht austreten kann.

In diesem Fall der Rückführung des Beatmungs-Rückgases zur Aufbereitung von neuem Beatmungsgas muss auch der Gehalt der Zielfraktion im Beatmungs-Rückgas oder nach dem Aufbereiten des neuen Beatmungsgases, aber vor einem zusätzlichen Zuführen von neuer Zielfraktion, bekannt sein und zu diesem Zweck mittel eines Sensors gemessen werden.

Der Ziel-Zufuhranschluss wird dann in Abhängigkeit vom Ergebnis des Sensors gesteuert, vorzugsweise mittels einer elektrischen oder elektronischen Steuerung, die vorzugsweise mit der Steuerung des übrigen Beatmungsgerätes gekoppelt ist oder in diese gar integriert ist.

Wenn die Zielfraktion nicht aktiv vom Rest des Beatmungsgases getrennt wird, ist ein Ziel-Abfuhranschluss für das Herausführen des Gemisches einschließlich der Zielfraktion in dem Teil des Gasweges angeordnet, der mit dem Reservoir und/oder dem Zufuhrregler für die Zielfraktion in Verbindung steht und dort ist vorzugsweise der Sensor für die Bestimmung an Zielfraktion angeordnet.

Dadurch ist eine Abtrennung der Zielfraktion von den übrigen Restfraktionen nicht notwendig, und es wird lediglich die notwendige zusätzliche Menge an Zielfraktion in den Gasweg des Gesamtgemisches, beispielsweise in einer dafür ausgebildeten Mischkammer, zugeführt.

Hierfür kann mittels des Zielzufuhr- und Zielabfuhranschlusses ein Bypass zum Hauptgasweg gebildet werden, der für das Zusetzen an weiterer Zielfraktion verwendet wird indem er mit dem Reservoir der Zielfraktion in Verbindung steht. Sofern das Rest-Gasgemisch nicht wieder aufbereitet sondern an die Umgebung entlassen wird (offener Kreislauf des Hauptgasgemisches) ist auf der vom Verbrauchspunkt abgewandten Seite des Zielzufuhr- und Zielabfuhranschlusses das Selektionselement, beispielsweise die selektive Membran, die alle Fraktionen außer der Zielfraktion durchlässt, notwendig.

Sofern das Hauptgasgemisch im Kreis geführt und wieder aufbereitet wird, ist ein solches Selektionselement unter Umständen nicht nötig, z. B. wenn
- dem Beatmungsrückgas bei der Wiederaufbereitung keine Fraktionen entnommen werden müssen und
- dem Rückgas neu zugesetzte Mengen über z. B. Ventile zugeführt werden, die einen Durchlass nur in Zufuhrrichtung in den Gaskreislauf hinein ermöglichen.

Um ein nachrüstbares Zusatzaggregat für ein herkömmliches Gasaufbereitungsgerät, wie etwa ein Beatmungsgerät, zu schaffen, ist es vor allem bei im Kreis geführtem Beatmungsgas als auch einer geschlossenen Kreislaufführung der Zielfraktion bzw. des Gasgemisches einschließlich der Zielfraktion möglich, Sensor, Zufuhrregler und auch den mit dem Zufuhrregler in Verbindung stehenden Speiseanschluss für das Reservoir an Zielfraktion, gegebenenfalls einschließlich des Reservoirs selbst und einem eventuell nicht notwendigen Druckerzeuger für die Zielfraktion in einer Ziel-Steuereinheit als geschlossene Baugruppe unterzubringen, die als Zieladapter in den Gasweg des Bearbeitungsgerätes montierbar ist und beispielsweise eine Bypassleitung bildet.

Sofern eine Selektionseinheit notwendig ist, ist vorzugsweise auch diese Selektionsvorrichtung, beispielsweise eine selektive Membran, in dem Adapter bzw. der Ziel-Steuereinheit angeordnet.

Bei einem y-förmigen Gasweg kann dies im Ast zwischen der Y-Stelle und dem Verbraucher, also dem Patienten, sein, aber auch in den aufgeteilten Ästen für Exspiration und Inspiration, in denen dann zumindest im Inspirationsast der Ziel-Zufuhranschluss angeordnet wird.

### c) Ausführungsbeispiele

Ausführungsformen gemäß der Erfindung sind im folgenden beispielhaft näher beschrieben. Es zeigen:
- Fig. 1:: eine Prinzipdarstellung der Vorgehensweise,
- Fig. 2:: mehrere allgemeine Möglichkeiten zur Lösung den Problems und
- Fig. 3:: konkrete Bauformen gemäß der Erfindung.

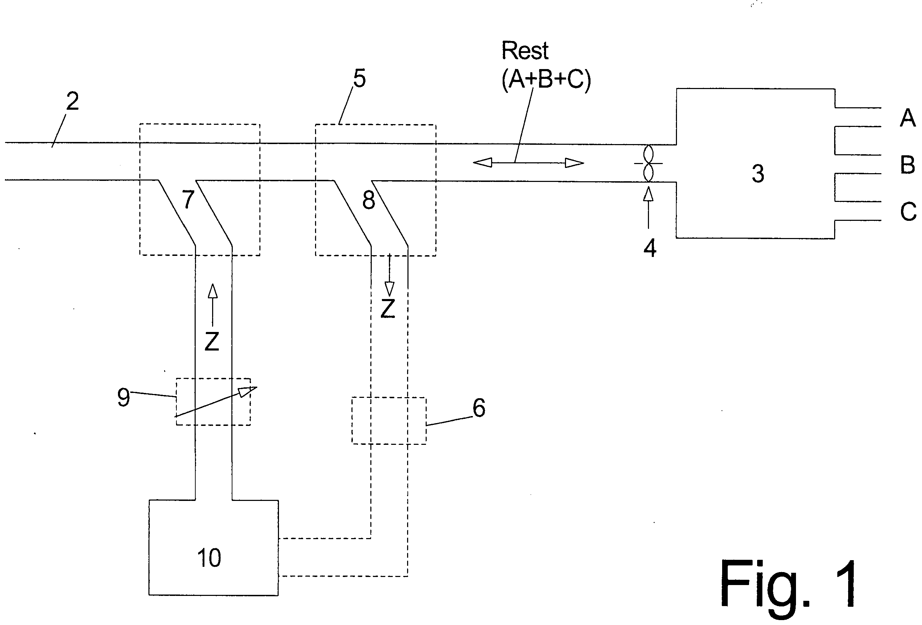

Fig. 1 zeigt grundsätzlich, wie in einer Mischkammer 3 aus mehreren Grundkomponenten A, B, C ein Gasgemisch erzeugt und über eine Leitung, in diesem Fall einem Intubations-Schlauch 2, einem Verbraucher, z. B. dem Patienten, zugeführt wird. Dazu ist ein Druckerzeuger 4, beispielsweise ein Gebläse, in der Leitung integriert. Diese Leitung soll zusätzlich über einen Ziel-Zufuhranschluss 7 eine weitere Zielkomponente Z zugeführt werden, was aus einem Reservoir 10) erfolgt, welches mit dem Zufuhranschluss 7 über einen steuerbaren Zufuhrregler 9 erfolgt.

Das Gasgemisch wird dabei über die Leitung 2 dem Verbraucher nicht nur zugeführt, sondern von diesem auch der unverbrauchte Rest, also das BeatmungsRückgas, auch wieder zurück in Richtung Mischkammer 3 abgegeben.

Damit hierbei die im Beatmungs-Rückgas enthaltene Zielfraktion Z nicht in die Mischkammer 3 und von dort über die Komponenten-Zuführungen nach außen verloren geht, ist im Verlauf des Gasweges ein Selektionselement 5 notwendig.

Dies kann eine aktive Trennvorrichtung zum Abtrennen der Komponente Z aus dem Gasgemisch sein, wodurch die Zielkomponente Z in das Reservoir rückgeführt oder anderweitig wieder verwertet werden kann. Dies kann auch lediglich eine passive Barriere sein, die ein Zurückfließen der Zielkomponente in die Komponentenzuflüsse A, B, C vermeidet.

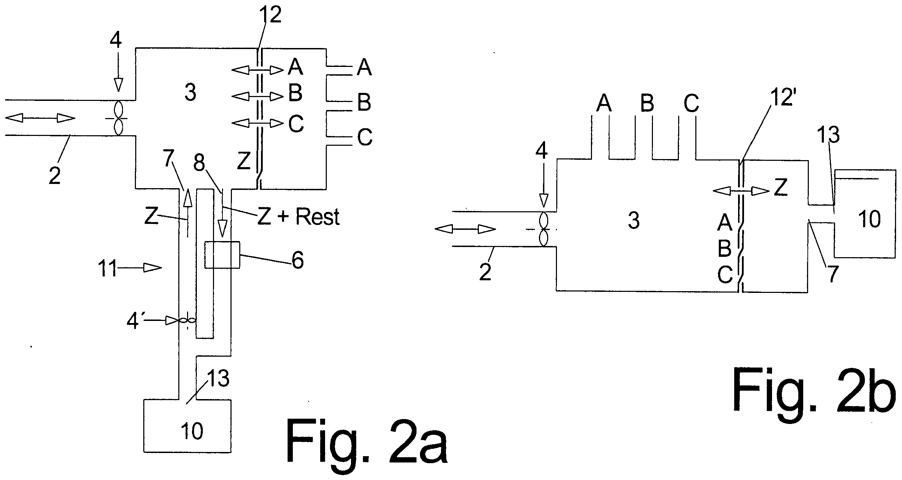

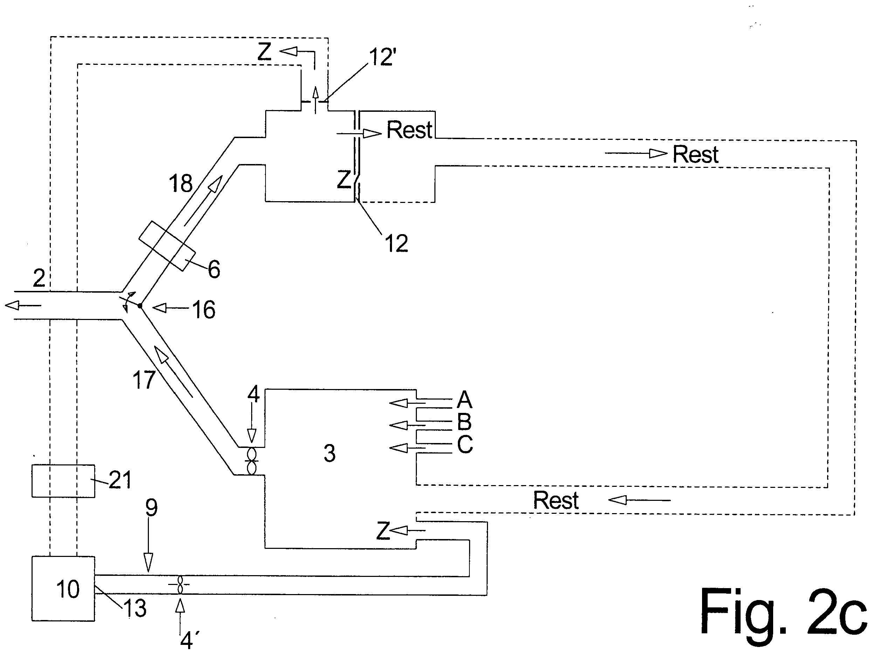

Wie Fig. 2 anzeigt, kann zu diesem Zweck, vorzugsweise direkt in die Mischkammer 3 hinein, die Zielkomponente Z aus dem Reservoir 10 dem Gemisch zugeführt werden, vorzugsweise wieder mittels Druckaufbau durch einen Druckerzeuger 4' zwischen Reservoir 10 und dem Zufuhranschluss 7.

Der Gehalt an Zielfraktion im Gasgemisch kann entweder in der Mischkammer 3 oder in der Zufuhrleitung, z. B. dem Intubations-Schlauch 2 zur Mischkammer gemessen werden oder auch in dem über einen Ziel-Abfuhranschluss 8 das Gesamtgemisch entnommen und über einen Bypass dem Zufuhranschluss 7 nach Ergänzen um zusätzliche Zielkomponente Z aus dem Reservoir 10 wieder zugeführt wird, wobei vor dem Zuführen im Bypass mittels eines Sensors 6 der Gehalt an Zielfraktion Z gemessen wird.

In die Mischkammer 3 münden auch die Zufuhranschlüsse für die weiteren Komponenten A, B, C, die für die Aufbereitung dem Beatmungsrückgas zum Teil zusätzlich zugegeben werden müssen. Ein Abfließen der Zielfraktion Z durch diese Zufuhranschlüsse A, B, C wird vermieden, in dem diese entweder hinter einer Membran 12 liegen, die für die Komponente Z nicht durchgängig ist, oder in dem die Zufuhranschlüsse für die Komponenten A, B, C so ausgebildet sind, dass sie nur in Zufuhrrichtung in die Gasmischkammer 3 hinein durchströmt werden können, egal von welcher Fraktion.

Fig. 2b zeigt im Gegensatz dazu eine Lösung, in der die Mischkammer eine Membran 12 aufweist, die nur für die Zielfraktion Z durchlässig ist und für die übrigen Komponenten A, B, C undurchlässig ist.

Dementsprechend befindet sich der Ziel-Zufuhranschluss 7, der mit dem Ziel-Speiseanschluss 13 und insbesondere einem Reservoir 10 hierfür in Verbindung steht, auf der vom Auslass, also dem Intubations-Schlauch 2 gegenüberliegenden Seite der Membran 12' in der Mischkammer, während die Anschlüsse A, B, C für die weiteren Komponenten sich auf der gleichen Seite der Membran wie der Auslassanschluss befinden.

Fig. 2c zeigt eine Lösung, bei der Inspirationspfad 17 und Expirationspfad 18 mit den entsprechenden Schläuchen ab dem Y-Stück 16 getrennt verlaufen, vorzugsweise mit einer Klappe zur wahlweisen Freigabe eines der beiden Schläuche zum Zentralstück hin, dem Intubations-Schlauch.

Im Beispiel der Fig. 2c findet vom Expirations-Schlauch 18 aus eine Abtrennung der Zielfraktion Z mittels einer Membran 12' statt, während der Rest des Beatmungs-Rückgases entweder an die Umgebung entlassen wird, dann jedoch über eine für die Z-Fraktion nicht durchlässige Membran 12, oder im Kreis zurückgeführt und der Gasmischkammer 3 zur Aufbereitung neuen Beatmungsgases zugeführt wird, wobei dann die Membran 12 nicht benötigt wird.

In die Mischkammer für das neue Beatmungsgas wird aus einem Reservoir 10 wiederum über einen steuerbaren Zufuhrregler 9 zusätzlich die Zielfraktion Z zugeführt, wofür gegebenenfalls ein Druckerzeuger 4' in dieser Zufuhrleitung benötigt wird.

In das Reservoir 10 kann auch das aus dem Exspirationspfad 17 abgezogene Zielfraktion Z eingespeist werden, wobei dann vorzugsweise seine Reinigungsvorrichtung 21 in diesem Rückführpfad angeordnet ist.

In diesem Fall wird also das Beatmungsgas bzw. Beatmungs-Rückgas wahlweise in einem offenen oder geschlossenen Kreislauf geführt, und auch die abgetrennte Zielfraktion Z wahlweise in einem geschlossenen Kreis geführt oder trotz offenen Kreislaufs einer Wiederverwertung zugeführt.

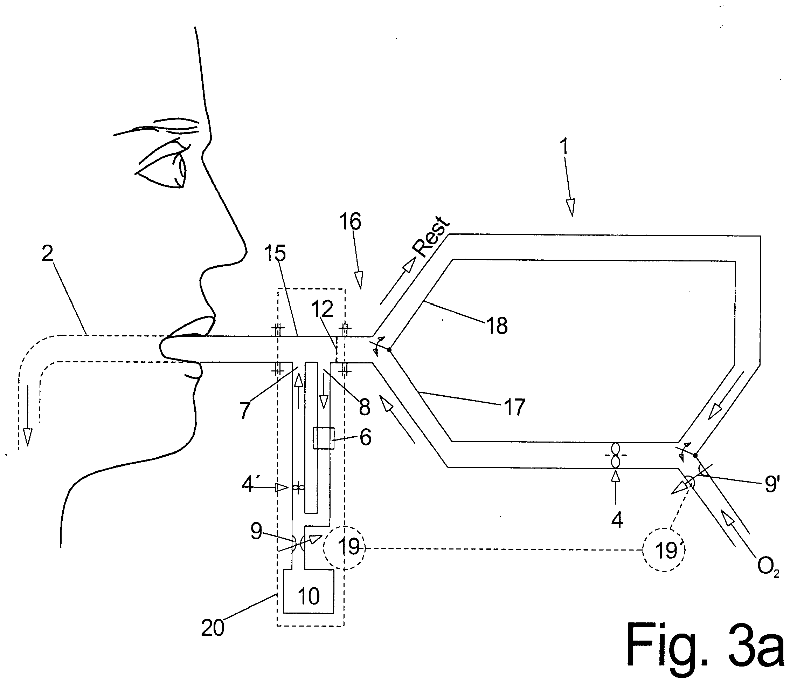

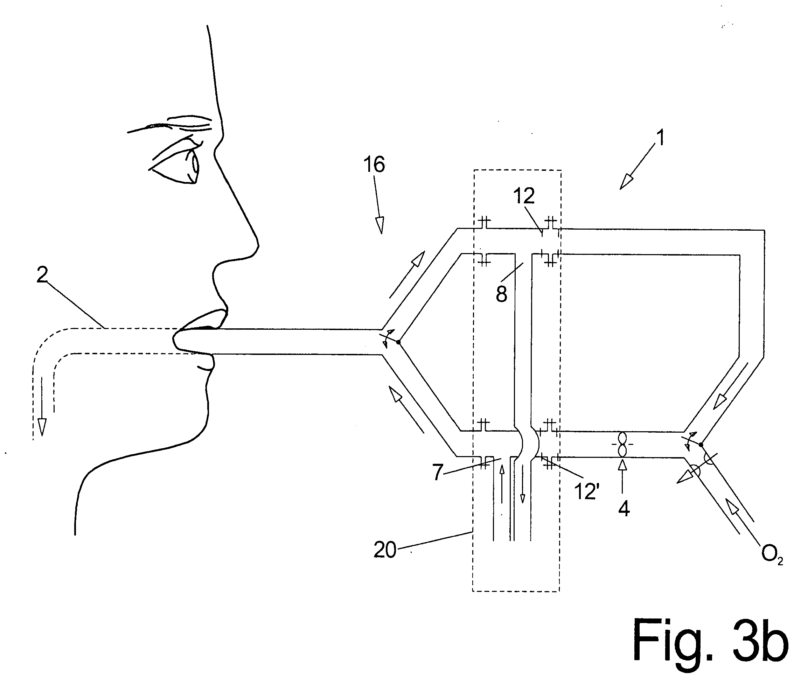

Die Fig. 3 zeigen eine konkrete Anwendung einer Applikator-Einheit 20, wie sie bei einem konventionellen Beatmungsgerät 1, welches den Patienten über ein Y-Stück 16 und einen Intubations-Schlauch 2 beatmet, nachträglich eingesetzt werden kann.

Dabei wird von einem geschlossenen Kreislauf des Beatmungsgases bzw. des Beatmungs-Rückgases ausgegangen, dem für die Wiederaufbereitung des Beatmungsgases aus dem Rückgas zusätzlich Sauerstoff zugesetzt wird über einen gesteuerten Zufuhrregler 9 und - was hier nicht dargestellt ist - gegebenenfalls auch CO₂ entzogen wird.

Gemäß Fig. 3a ist die Applikator-Einheit 20 zwischen dem Intubations-Schlauch 2 und dem Y-Stück 16 angeordnet. Die Applikator-Einheit 20 umfasst dabei einen Zieladapter 15, der als Zwischenstück zwischen Intubations-Schlauch 2 und Y-Stück 16 montiert wird und von welchem ein Bypass 11 analog der Prinzipdarstellung der Fig. 2a abzweigt, also mit einem Sensor 6 zur Ermittlung des Gehaltes an Zielfraktion in dem Gasgemisch, einer Zufuhr an Zielfraktion über einen geregelten Zufuhrregler 9 aus einem Reservoir 10 der Zielfraktion und soweit notwendig mittels Druckaufbau durch einen in der Bypassleitung angeordneten Druckerzeuger 4'.

Der Zufuhrregler 9 wird angesteuert von einer Steuerung 19, die die Signale des Sensors 6 erhält und vorzugsweise in Verbindung steht mit der Steuerung 19' des Beatmungsgerätes 1 oder gar in diese integriert ist.

Die zwischen den Zufuhr- und Abfuhranschlüssen 7 und 8 für die Zielfraktion Z und dem Y-Stück 16 eingezeichnete Membran 12, die für alle Fraktionen außer die Zielfraktion Z durchlässig ist, verhindert ein Einströmen der Zielfraktion Z in den restlichen Kreislauf des Beatmungsgases. Dies ist nur notwendig, wenn die sonstigen Ein- und Auslässe dieses Kreislaufes ein Ausströmen der Z-Komponente aus dem Kreislauf ermöglichen würden. Wenn dies durch spezielle Einlassventile für z. B. den Sauerstoff ausgeschlossen ist, kann auf die Membran 12 auch verzichtet werden.

Fig. 3b zeigt eine Lösung, bei der der Ziel-Zufuhranschluss 7 und der Ziel-Abfuhranschluss 8 für die Zielkomponente Z auf der patientenabgewandten Seite des Y-Stückes 16 angeordnet sind, und zwar in der Expirationsleitung 18 der Ziel-Abfuhranschluss 8 und in der Inspirationsleitung 17 der Ziel-Zufuhranschluss 7, wenn die Zielfraktion Z zusammen mit dem Rest auch hier im Bypass 11 im geschlossenen Kreis geführt wird.

Auch dann ist in jedem eine Membran 12 bzw. 12' notwendig, sofern der Rest des Kreislaufes des Beatmungsgases, z. B. die Einspeisung für den Sauerstoff, nicht konstruktionsbedingt ein Abfließen der Zielfraktion verhindert. Ist dies jedoch gegeben, kann auch hier auf die Membranen 12, 12' verzichtet werden.

Dann kann jedoch unter Umständen auf den Ziel-Abfuhranschluss 8 ebenfalls komplett verzichtet werden, sofern der Sensor 6 stattdessen direkt im Kreislauf des Beatmungsgases vorzugsweise unmittelbar vor dem Ziel-Zufuhranschluss 7, angeordnet wird.

Der Ziel-Zufuhranschluss 7 steht dann lediglich mit dem Reservoir 10 über dem steuerbaren Zufuhrregler 9 in Verbindung.

### BEZUGSZEICHENLISTE

- 1: Beatmungsgerät
- 2: Intubations-Schlauch
- 3: Gas-Mischkammer
- 4, 4': Druckerzeuger
- 5: Selektionselement
- 6: Sensor
- 7: Ziel-Zufuhr-Anschluss
- 8: Ziel-Abfuhr-Anschluss
- 9: Zufuhr-Regler
- 10: Reservoir
- 11: Bypass
- 11a, b: Bypassleitungen
- 12, 12': Membran
- 13: Ziel-Speiseanschluss
- 14: Ziel-Steuereinheit
- 15: Ziel-Adapter
- 16: Y-Stück
- 17: Inspirations-Schlauch
- 18: Exspirations-Schlauch
- 19: Steuerung
- 20: Applikator-Einheit
- 21: Reinigungsvorrichtung

## Patentansprüche

1. Beatmungsgerät (1) mit
- einem Intubationsschlauch (2),
- einer Gasmischkammer (3) und einem Druckerzeuger (4),
**dadurch gekennzeichnet, dass**
- zwischen Intubationsschlauch (2) und Gasmischkammer (3) ein zur Wiederverwertung der Zielfraktion geeignetes Selektionselement (5) als passive Trennvorrichtung zum Abtrennen der Ziefraktion (Z) von den restlichen Fraktionen des Beatmungs-Rückgases vorhanden ist,
- ein Ziel-Zufuhranschluss (7), der mit einem gesteuerten Zufuhrregler (9) mit einem Ziel-Fraktion-Reservoir (10) in Verbindung steht,
- wenigstens ein Sensor (6) zur Bestimmung der Konzentration der Ziel-Fraktion (Z) in dem Gasweg des Beatmungsrückgases des Beatmungsgerätes vorhanden ist
- der Sensor (6) den Zufuhrregler (9) steuert,
- das Selektionselement (5) als eine passiv wirkende selektive Sperre ausgebildet ist und die selektive Sperre eine Membran (12) aufweist, die nur für die Zielfraktion (Z) durchlässig ist und für die übrigen Komponenten (A, B, C) undurchlässig ist.

2. Beatmungsgerät (1) nach Anspruch 1,
**dadurch gekennzeichnet, dass**
die Membran (12) für die Zielfraktion einerseits und die restlichen Fraktionen des Gasgemisches andererseits, gegebenenfalls in Abhängigkeit zu einer Durchtrittsrichtung, eine unterschiedliche Permeabilität aufweist.

3. Beatmungsgerät (1) nach einem der vorhergehenden Ansprüche,
**dadurch gekennzeichnet, dass**
mit dem Ziel-Zufuhr-Anschluss (7) ein Druckerzeuger (4') in Verbindung steht.

4. Beatmungsgerät (1) nach einem der vorhergehenden Ansprüche,
**dadurch gekennzeichnet, dass**
ein Ziel-Abfuhr-Anschluss (8) in dem Teil des Gasweges des Beatmungsgases vorhanden ist, der mit dem Reservoir (10) und/oder dem Zufuhr-Regler (9) in Verbindung steht und insbesondere den Sensor (6) enthält, wodurch ein mit dem Reservoir (10) in Verbindung stehender Bypass (11), insbesondere für das Gesamtgemisch gebildet wird.

5. Beatmungsgerät (1) nach Anspruch 2,
**dadurch gekennzeichnet, dass**
die selektive Membran (12), insbesondere in beide Richtungen, nur für die Ziel-Fraktion (Z) durchlässig ist, nicht jedoch für die übrigen Fraktionen des Gasgemisches, und der Ziel-Abfuhr-Anschluss (8) auf einer Ziel-Fraktion-Reinseite der Membran (12) angeordnet ist.

6. Beatmungsgerät (1) nach einem der Ansprüche 2bis 4,
**dadurch gekennzeichnet, dass**
die Membran (12) für alle anderen Fraktionen außer der Ziel-Fraktion (Z) des Gasgemisches durchlässig ist, und diese Eigenschaft insbesondere für beide Durchströmungsrichtungen der Membran gilt, und Zufuhranschluss (7) sowie Abfuhr-Anschluss (8) auf einer Ziel-Gemisch-Seite der Membran (12) münden.

7. Beatmungsgerät (1) nach einem der Ansprüche 2 bis 4,
**dadurch gekennzeichnet, dass**
Sensor (6), Regler (9) sowie wenigstens ein Ziel-Speise-Anschluss (13) für das Reservoir (10), insbesondere einschließlich des Reservoirs (10), ggf. auch ein Ziel-Druckerzeuger (4'), in einer Ziel-Steuereinheit (14) als geschlossene Baugruppe untergebracht sind, die über Bypass-Leitungen (11a, b) mit dem Beatmungsgerät (1) verbindbar ist.

8. Beatmungsgerät (1) nach Anspruch 7,
**dadurch gekennzeichnet, dass**
das Beatmungsgerät (1) im Gasweg einen Ziel-Adapter (15) zum Anschließen der Ziel-Steuereinheit (14) umfasst, der die selektive Membran (12) enthält und als Zwischenstück zwischen Intubationsschlauch (2) und Gasmischkammer (3) einsatzbar ist, bei einem Y-förmigen Pfad insbesondere stromabwärts des Y-Stückes (16) im Intubations-Schlauch (2).

9. Beatmungsgerät (1) nach Anspruch 8,
**dadurch gekennzeichnet, dass**
der Ziel-Adapter (15) zusammen mit den Bypass-Leitungen (11a, b) und der Ziel-Steuereinheit (14) eine einheitliche, insbesondere nachrüstbare, Applikator-Einheit (20) bilden.

10. Beatmungsgerät (1) nach einem der vorhergehenden Ansprüche,
**dadurch gekennzeichnet, dass**
der Zufuhr-Regler (9) sowie ggf. der Sensor (6) mit einer insbesondere elektrischen oder elektronischen Steuerung (19) gekoppelt ist, die gemeinsam in der Lage sind, eine konstante Konzentration der Zielfraktion (Z) im Gemisch des Beatmungsgases einzuhalten.

11. Beatmungsgerät (1) nach einem der vorhergehenden Ansprüche,
**dadurch gekennzeichnet, dass**
die Ziel-Fraktion (Z) ein Edelgas, insbesondere Xenon, ist.

12. Beatmungsgerät (1) nach Anspruch 10 oder 11,
**dadurch gekennzeichnet, dass**
die Steuerung (19) für die Ziel-Fraktion (Z) mit der Steuerung (19') des Beatmungsgerätes (1) gekoppelt, insbesondere in diese integriert, ist.

13. Verfahren zur Wiederverwendung und Zumischung einer Ziel-Gas-Fraktion in einem Gasgemisch, zur Verwendung außerhalb der therapeutischen Anwendung,
**dadurch gekennzeichnet, dass**
- das, in einer Gasmischkammer mit einer Membran versehene, enthaltene Gasgemisch über den Verbraucher im Kreislauf geführt wird, wobei die Membran (12) nur für die Zielfraktion (Z) durchlässig ist und für die übrigen Komponenten (A,B,C) undurchlässig ist,
- die Konzentration der Zielfraktion im Gemisch an mindestens einer Stelle des Kreislaufes gemessen, insbesondere ständig gemessen, wird, und
- durch Vergleich der Soll-Konzentration mit der Ist-Konzentration die Zufuhr von zusätzlicher Ziel-Fraktion gesteuert wird.

14. Verfahren nach Anspruch 13,
**dadurch gekennzeichnet, dass**
das Messen der Ist-Konzentration der Zielgasfraktion unmittelbar in einem Beatmungsrückgas erfolgt.

15. Verfahren nach einem der Ansprüche 13 oder 14,
**dadurch gekennzeichnet, dass**
das Messen der Ist-Konzentration der Zielgasfraktion in einem Bypass zum Herausführen des Gemisches und Zuführen des Gemisches mit der zugesetzten Zielfraktion aus dem Hauptkreislauf erfolgt.

16. Verfahren nach einem der Ansprüche 13 bis 15,
**dadurch gekennzeichnet, dass**
das Gasgemisch in einem offenen Kreislauf geführt wird und die Ziel-Gas-Fraktion vor dem Auslauf des offenen Kreislaufes vom Rest des Gemisches separiert und dem Zielgas-Reservoir insbesondere für die Zielgaszufuhr mit Zeitversatz, insbesondere bedingt durch Wiederaufbereitung und Komprimierung, zugeführt wird.

## Claims

1. Respirator (1) comprising
- an intubation hose (2),
- a gas mixing chamber (3) and a pressure generator (4),
**characterised in that**
a selection element (5) suitable for recycling the target fraction is provided between the intubation tube (2) and the gas mixing chamber (3) as a passive separating device for separating the target fraction (Z) from the remaining fractions of return breathing gas,
a target supply connection (7) is connected to a target-fraction reservoir (10) via a controlled supply regulator (9),
at least one sensor (6) for determining the concentration of the target fraction (Z) is provided in the gas route of the return breathing gas of the respirator,
- the sensor (6) controls the supply regulator (9), and
the selection element (5) is designed as a passive selective barrier, and the selective barrier comprises a membrane (12) which is permeable only to the target fraction (Z) and impermeable to the remaining components (A, B, C).

2. Respirator (1) according to claim 1, **characterised in that** the membrane (12) has different permeabilities for the target fraction and for the remaining fractions of the gas mixture, optionally depending on a direction of passage.

3. Respirator (1) according to either of the preceding claims, **characterised in that** a pressure generator (4') is connected to the target supply connection (7).

4. Respirator (1) according to any of the preceding claims, **characterised in that** a target discharge connection (8) is provided in the portion of the gas route of the breathing gas that is connected to the reservoir (10) and/or to the supply regulator (9) and in particular contains the sensor (6), as a result of which a bypass (11) connected to the reservoir (10), in particular for the total mixture, is formed.

5. Respirator (1) according to claim 2, **characterised in that** the selective membrane (12) is permeable only to the target fraction (Z), in particular in both directions, but is impermeable to the remaining fractions of the gas mixture, and the target discharge connection (8) is arranged on a target-fraction clean side of the membrane (12).

6. Respirator (1) according to any of claims 2 to 4, **characterised in that** the membrane (12) is permeable to all other fractions of the gas mixture except the target fraction (Z), which property applies in particular to both through-flow directions of the membrane, and the supply connection (7) and the discharge connection (8) lead onto a target-mixture side of the membrane (12).

7. Respirator (1) according to any of claims 2 to 4, **characterised in that** the sensor (6), the regulator (9) and at least one target feed-in connection (13) for the reservoir (10), which connection in particular comprises the reservoir (10), and optionally also a target pressure generator (4'), are housed in a target control unit (14) as a closed assembly which can be connected to the respirator (1) by means of bypass lines (11a, b).

8. Respirator (1) according to claim 7, **characterised in that** the respirator (1) comprises, in the gas route, in the region of a Y-shaped path and in particular downstream of the Y-portion (16) in the intubation tube (2), a target adaptor (15) for connecting the target control unit (14), which adaptor contains the selective membrane (12) and can be inserted as a coupling piece between the intubation tube (2) and the gas mixing chamber (3).

9. Respirator (1) according to claim 8, **characterised in that** the target adaptor (15), together with the bypass lines (11a, b) and the target control unit (14), forms a homogenous, in particular retrofittable, applicator unit (20).

10. Respirator (1) according to any of the preceding claims, **characterised in that** the supply regulator (9), and optionally also the sensor (6), is coupled to a controller (19), which is in particular electric or electronic, which regulator and controller are together capable of keeping the concentration of the target fraction (Z) constant in the mixture of breathing gases.

11. Respirator (1) according to any of the preceding claims, **characterised in that** the target fraction (Z) is a noble gas, in particular xenon.

12. Respirator (1) according to either claim 10 or claim 11, **characterised in that** the controller (19) for the target fraction (Z) is coupled to the controller (19') of the respirator (1), and in particular integrated therein.

13. Method for reusing and admixing a target-gas fraction in a gas mixture for use outside the therapeutic application, **characterised in that**
- the contained gas mixture, which is provided in a gas chamber comprising a membrane, is guided in a circular flow by the user, the membrane (12) being permeable only to the target fraction (Z) and impermeable to the remaining components (A, B, C),
- the concentration of the target fraction in the mixture is measured at at least one point in the circular flow, and is in particular measured continuously, and
- the supply of additional target fraction is controlled by comparing the desired concentration with the actual concentration.

14. Method according to claim 13, **characterised in that** the measurement of the actual concentration of the target-gas fraction is carried out directly in return breathing gas.

15. Method according to either claim 13 or claim 14, **characterised in that** the measurement of the actual concentration of the target-gas fraction is carried out in a bypass for guiding the mixture out and supplying the mixture comprising the added target fraction from the main circular flow.

16. Method according to any of claims 13 to 15, **characterised in that** the gas mixture is guided in an open circular flow and the target-gas fraction is separated from the rest of the mixture before the outlet of the open circular flow and supplied to the target-gas reservoir, in particular for supplying target gas with a time offset, in particular due to reprocessing and compression.

## Revendications

1. Respirateur (1) avec
- un tuyau d'intubation (2),
- une chambre de mélange de gaz (3) et un générateur de pression (4),
**caractérisé en ce que**
- entre le tuyau d'intubation (2) et la chambre de mélange de gaz (3) un élément de sélection (5) approprié pour la réutilisation de la fraction de destination est prévu en tant que dispositif de séparation passif pour séparer la fraction cible (Z) des autres fractions du gaz respiratoire de retour,
- un raccord d'alimentation cible (7) qui est en communication avec un régulateur d'alimentation réglé (9) avec un réservoir de fraction cible (10),
- au moins un capteur (6) est présent pour déterminer la concentration de la fraction cible (Z) dans le passage du gaz respiratoire de retour du respirateur,
- le capteur (6) commande le régulateur d'alimentation (9),
- l'élément de sélection (5) est adapté en tant que barrière agissant de manière passive sélective et la barrière sélective comprenant une membrane (12) qui n'est perméable que pour la fraction cible (Z) et qui n'est pas perméable pour les autres composants (A, B, C).

2. Respirateur (1) selon la revendication 1,
**caractérisé en ce que**
la membrane (12) pour la fraction cible d'un côté et les autres fractions du mélange de gaz de l'autre côté présente des perméabilités différentes, le cas échéant en dépendance d'un sens de passage.

3. Respirateur (1) selon une des revendications précédentes,
**caractérisé en ce que**
un générateur de pression (4') est en communication avec le raccord d'alimentation cible (7).

4. Respirateur (1) selon une des revendications précédentes,
**caractérisé en ce que**
un raccord de décharge cible (8) est présent dans la partie du passage de gaz du respirateur qui est en communication avec le réservoir (10) et/ou le régulateur d'alimentation (9) et comprend en particulier le capteur (6), formant ainsi un contournement (11) en communication avec le réservoir (10), en particulier pour le mélange total.

5. Respirateur (1) selon la revendication 2,
**caractérisé en ce que**
la membrane sélective (12) est perméable seulement pour la fraction cible (Z), en particulier dans les deux sens, mais non pas pour les autres fractions du mélange de gaz, et **en ce que** le raccord de décharge cible (8) est arrangé sur un côté fraction cible pure de la membrane (12).

6. Respirateur (1) selon une des revendications 2 à 4,
**caractérisé en ce que**
la membrane (12) est perméable pour toutes les autres fractions à part la fraction cible (Z) du mélange de gaz, et **en ce que** cette caractéristique vaut en particulier pour les deux sens de passage de la membrane, **en ce que** le raccord d'alimentation (7) et le raccord de décharge cible (8) débouchent sur un côté mélange cible de la membrane (12).

7. Respirateur (1) selon une des revendications 2 à 4,
**caractérisé en ce que**
le capteur (6), le régulateur (9) ainsi qu'au moins un raccord d'alimentation cible (13) pour le réservoir (10), en particulier le réservoir (10) inclus, le cas échéant aussi un générateur de pression cible (4') sont arrangés dans une unité de commande cible (14) en tant que module fermé qui peut être lié au respirateur (1) par des conduits de contournement (11a, b).

8. Respirateur (1) selon la revendication 7,
**caractérisé en ce que**
le respirateur (1) comprend dans le passage de gaz un adaptateur cible (15) pour connecter l'unité de commande cible (14) qui comprend la membrane séléctive (12) et qui peut être introduite en tant que pièce intermédiaire entre le tuyau d'intubation (2) et la chambre de mélange de gaz (3), pour un passage en forme de Y, en particulier en aval du pièce en Y (16) dans le tuyau d'intubation (2).

9. Respirateur (1) selon la revendication 8,
**caractérisé en ce que**
l'adaptateur cible (15) forme avec les conduits de contournement (11a, b) et l'unité de commande cible (14) une unité d'applicateur (20) uniforme, en particulier à installer ultérieurement.

10. Respirateur (1) selon une des revendications précédentes,
**caractérisé en ce que**
le régulateur d'alimentation (9) et le cas échéant le capteur (6) sont couplés à une commande électrique ou électronique (19) qui ensemble sont capables de maintenir une concentration constante de la fraction cible (Z) dans le mélange du gaz respiratoire.

11. Respirateur (1) selon une des revendications précédentes,
**caractérisé en ce que**
la fraction cible (Z) est un gaz noble, en particulier du xénon.

12. Respirateur (1) selon une des revendications 10 où 11,
**caractérisé en ce que**
la commande (19) pour la fraction cible (Z) est couplée à la commande (19') du respirateur (1), et est en particulier intégré dans celle-ci.

13. Procédé pour la réutilisation et l'ajout d'une fraction de gaz cible dans un mélange de gaz pour l'utilisation en dehors de l'application thérapeutique,
**caractérisé en ce que**
- le mélange de gaz pourvu d'une membrane dans une chambre de mélange de gaz est guidé via le consommateur dans le circuit,
la membrane (12) n'étant perméable que pour la fraction cible (Z) et n'étant pas perméable pour les autres composants (A, B, C),
- la concentration de la fraction cible dans le mélange est mesurée en particulier de manière continue dans au moins un endroit dans le circuit, et
- l'apport de fraction cible supplémentaire est réglé par la comparaison de la concentration cible à la concentration réelle.

14. Procédé selon la revendication 13,
**caractérisé en ce que**
la mesure de la concentration réelle de la fraction de gaz cible est réalisé directement dans un gaz respiratoire de retour.

15. Procédé selon une des revendications 13 ou 14,
**caractérisé en ce que**
la mesure de la concentration réelle de la fraction de gaz cible est réalisé dans un contournement pour sortir le mélange et pour apporter le mélange avec la fraction cible ajoutée du circuit principal.

16. Procédé selon une des revendications 13 à 15,
**caractérisé en ce que**
le mélange de gaz est guidé dans un circuit ouvert et **en ce que** la fraction de gaz cible est séparée du reste du mélange avant la sortie du circuit ouvert et est amené au réservoir de gaz cible en particulier pour l'apport de gaz cible avec un décalage dans le temps, en particulier à cause d'un recyclage et d'une compression.
